(19) Europäisches Patentamt
European Patent Office
Office européen des brevets

(11) EP 3 782 973 B1

(12) **EUROPÄISCHE PATENTSCHRIFT**

(45) Veröffentlichungstag und Bekanntmachung des Hinweises auf die Patenterteilung:
**01.03.2023 Patentblatt 2023/09**

(21) Anmeldenummer: **19020479.2**

(22) Anmeldetag: **19.08.2019**

(51) Internationale Patentklassifikation (IPC):
***C07C 29/151*** *(2006.01)* ***C07C 31/04*** *(2006.01)*
***B01J 8/04*** *(2006.01)* ***B01J 12/00*** *(2006.01)*

(52) Gemeinsame Patentklassifikation (CPC):
(C-Sets verfügbar)
**B01J 8/04; B01J 12/007; C07C 29/1518;**
B01J 2208/00017; B01J 2208/00628 (Forts.)

(54) **VERFAHREN UND ANLAGE ZUR HERSTELLUNG VON METHANOL**

PROCESS AND SYSTEM FOR THE MANUFACTURE OF METHANOL

PROCÉDÉ ET INSTALLATION DE FABRICATION DE MÉTHANOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**24.02.2021 Patentblatt 2021/08**

(73) Patentinhaber: **L'AIR LIQUIDE, SOCIÉTÉ ANONYME POUR L'ÉTUDE ET L'EXPLOITATION DES PROCÉDÉS GEORGES CLAUDE**
**75007 Paris (FR)**

(72) Erfinder: **Schuhmann, Timm**
**64625 Bensheim (DE)**

(74) Vertreter: **Stang, Stefan**
**Air Liquide Forschung und Entwicklung GmbH**
**Gwinnerstraße 27-33**
**60388 Frankfurt am Main (DE)**

(56) Entgegenhaltungen:
**WO-A1-2015/193440**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



**(Forts. nächste Seite)**

(52) Gemeinsame Patentklassifikation (CPC): (Forts.)

C-Sets
**C07C 29/1518, C07C 31/04**

## Beschreibung

### Gebiet der Erfindung

Die Erfindung betrifft ein Verfahren zur Herstellung von Methanol, eine Anlage zur Herstellung von Methanol sowie die Verwendung der erfindungsgemäßen Anlage im erfindungsgemäßen Verfahren.

### Stand der Technik

Methanol wird heute üblicherweise durch katalytische Umsetzung von Synthesegas, einem Gemisch aus vorwiegend Wasserstoff ($H_2$), Kohlenmonoxid (CO) sowie Kohlendioxid ($CO_2$) im sogenannten Niederdruckverfahren bei Drücken zwischen 60 und 120 bar hergestellt. Kohlenmonoxid und Kohlendioxid können zusammengefasst auch als "Kohlenstoffoxide" bezeichnet werden.

Je nach Technologie und Anlagenkapazität kommen bezogen auf die Reaktorsektion des Methanol-Herstellungsverfahrens meist entweder einstufige oder zweistufige Verfahren zur Anwendung, um das Synthesegas mit hoher Ausbeute zu Methanol umzusetzen.

Bei den verwendeten Verfahren wird das Synthesegas, welches meist bei Drücken zwischen 20 und 40 bar erzeugt wird, zunächst über einen Gaskompressor (Synthesegaskompressor) auf den für die Reaktion erforderlichen Druck (Synthesedruck) von größer als 60 bar gebracht. Die Verfahren arbeiten ferner meist mit hohen Gaskreisläufen in einem sogenannten Synthesekreislauf, auch als Syntheseloop bezeichnet, in dem nicht im ersten Reaktordurchgang (per-pass) zu Methanol umgesetztes Synthesegas als Rückführgas, auch als Recyclegas bezeichnet, zum Reaktoreingang zurückgeführt wird. Der Synthesekreislauf ist erforderlich, um trotz der niedrigen per-pass Umsätze ausreichend hohe Synthesegasumsätze und damit Gesamtausbeuten zu erzielen.

Die Rückführgasmenge liegt dabei je nach Technologie, Frischgaszusammensetzung und Reaktorsystem bis zu 5 mal höher als die zugeführte Frischgasmenge aus der Synthesegaserzeugung.

Ein Nachteil dieser hohen Rezirkulationsraten ist die Akkumulation von inerten, das heißt unter den Bedingungen der Methanolsynthese nicht umsetzbaren Bestandteilen wie Methan und Stickstoff, welche als Spülgas, auch als Purgegas bezeichnet, aus dem Synthesekreislauf zu entfernen sind. Dabei wird üblicherweise auch Wasserstoff entfernt, welcher anschließend aufwändig zurückgewonnen werden muss, was meist mit Hilfe einer Wechseldruckabsorptionsvorrichtung geschieht.

Weiterhin müssen die einzelnen Komponenten der betreffenden Produktionsanlage bei der Verwendung eines Synthesekreislaufs für die großen Rückführgasflüsse entsprechend dimensioniert werden. Dies hat zur Folge, dass die gesamten Investitionskosten (CAPEX) der Anlage ansteigen und/oder bei hohen erforderlichen Kapazitäten die Abmessungen der Reaktoren, insbesondere der äußere Durchmesser, an die Transportlimitierungen des jeweiligen Anlagenprojekts kommen können.

Weiterhin wird für die Rückverdichtung des Rückführgases auf Synthesedruck eine zusätzliche, dedizierte Kompressor-Einheit zur Verdichtung des Rückführgases (Rückführgas- oder Recyclegas-Kompressor) benötigt, womit nicht nur die Investitionskosten (CAPEX) erhöht werden, sondern auch die gesamte erforderliche Kompressorleistung und damit die Betriebskosten der Anlage (OPEX).

Beispiele für Verfahren mit Synthesekreislauf sind in der WO 2015/193440 A1, der WO 2017/167642 A1 sowie der EP 2 116 295 A1 offenbart.

Durch das Hinzufügen von zusätzlichen Reaktorstufen inklusive Zwischenkondensation des je Reaktorstufe erzeugten Methanols kann der per-pass Umsatz über die Gesamtzahl der Reaktorstufen erhöht werden und dadurch die Rezirkulationsrate verringert werden. Dadurch wird zwar der Anteil des Rückführgases (Recycle-Gases) verringert, gleichzeitig erhöht sich jedoch die Anzahl der erforderlichen Prozesseinheiten, wodurch die Investitionskosten (CAPEX) steigen. Wird auf einen Synthesekreislauf vollständig verzichtet ("once-through"-Prozess), so erhöht sich die Anzahl der erforderlichen Reaktoren weiter, so dass der Verzicht auf den Synthesekreislauf wiederum negativ kompensiert wird.

### Beschreibung der Erfindung

Im Hinblick auf die Herstellung von Methanol aus Kohlenstoffoxiden und Wasserstoff aufweisendem Synthesegas besteht somit Bedarf für die Verbesserung bestehender Verfahren.

Eine Aufgabe der vorliegenden Erfindung besteht darin ein Verfahren zur Herstellung von Methanol bereitzustellen, welches die Nachteile des Standes der Technik zumindest teilweise überwindet.

Eine weitere Aufgabe der vorliegenden Erfindung besteht darin ein Verfahren zur Herstellung von Methanol bereitzustellen, welches Einsparungen bezüglich der für das Verfahren aufzuwendenden Energie, insbesondere zum Betrieb von Gaskompressoren, ermöglicht.

Eine weitere Aufgabe der vorliegenden Erfindung besteht darin ein Verfahren zur Herstellung von Methanol

bereitzustellen, welches Einsparungen bezüglich der für das Verfahren bereitzustellenden Anlagenteile, insbesondere im Hinblick auf die Zahl erforderlicher Kompressorstufen, ermöglicht.

Eine weitere Aufgabe der vorliegenden Erfindung besteht darin ein Verfahren zur Herstellung von Methanol bereitzustellen, welches Wasserstoff-Verluste über einen Purgegas-Strom (Spülgas-Strom) weitestgehend vermeidet.

Eine weitere Aufgabe der vorliegenden Erfindung besteht darin ein Verfahren zur Herstellung von Methanol bereitzustellen, welches die Katalysator-Belastung durch einen hohen Wasser-Partialdruck im Katalysatorbett verringert und die Lebensdauer der verwendeten Katalysatoren verlängert.

Eine weitere Aufgabe der vorliegenden Erfindung besteht darin ein Verfahren zur Herstellung von Methanol bereitzustellen, welches die Menge an in einem Synthesekreislauf rückzuführenden Synthesegas so weit wie möglich verringert.

Eine weitere Aufgabe der vorliegenden Erfindung besteht darin eine Anlage zur Herstellung von Methanol bereitzustellen, welche zumindest eine der vorgenannten Aufgaben zumindest teilweise löst.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie.

Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Die vorgenannten Aufgaben werden zumindest teilweise gelöst durch ein Verfahren zur Herstellung von Methanol, umfassend folgende Prozessschritte, wobei diese nicht zwingend in der angegebenen Reihenfolge auszuführen sind:

a) Bereitstellen eines Kohlenstoffoxide ($CO$, $CO_2$) und Wasserstoff ($H_2$) aufweisenden Einsatzgases;
b) Einschleusen des Einsatzgases als einen ersten Frischgasstrom in eine erste Kompressorstufe zur Vorverdichtung des ersten Frischgasstroms, wobei ein zweiter Frischgasstrom erhalten wird;
c) Einschleusen eines Rückführgasstroms und des zweiten Frischgasstroms in eine zweite Kompressorstufe zur Verdichtung des Rückführgasstroms und des zweiten Frischgasstroms auf Synthesedruck, wobei ein Synthesegasstrom erhalten wird;
d) Katalytisches Umsetzen des Synthesegases des Synthesegasstroms in einer Mehrzahl in Serie angeordneter Reaktorstufen bei Synthesedruck, wobei je Reaktorstufe ein Methanol und nicht reagiertes Synthesegas aufweisender Produktstrom erhalten wird;
e) Kühlung des je Reaktorstufe erhaltenen Produktstroms zur Kondensation und Abtrennung von Methanol von nicht reagiertem Synthesegas und Einschleusen von nicht reagiertem Synthesegas in eine jeweils folgende der in Serie angeordneten Reaktorstufen;
f) Abziehen von nicht reagiertem Synthesegas aus zumindest einer der Reaktorstufen als Rückführgasstrom zum Einschleusen des Rückführgasstroms in die zweite Kompressorstufe gemäß Schritt c).

Erfindungsgemäß wird das Rückführgas zusammen mit dem vorverdichteten zweiten Frischgasstrom in die zweite Kompressorstufe eingeschleust. Durch die katalytische Umsetzung des Synthesegases in einer Mehrzahl in Serie angeordneter Reaktorstufen wird die Rückführgasmenge aufgrund der hohen per-pass Umsätze über alle Reaktorstufen reduziert. Dadurch kann aus einer oder mehreren Reaktorstufen abgezogener Rückführgasstrom unmittelbar der zweiten Kompressorstufe (zusammen mit dem zweiten Frischgasstrom) zugeführt werden, wodurch auf einen Rückführgaskompressor verzichtet werden kann. Ferner sinkt überraschend die für das Verfahren gesamte erforderliche Kompressorleistung.

Je Reaktorstufe wird Synthesegas zu einem Produktstrom umgesetzt, welcher Methanol aufweist. Dabei handelt es sich um ein Rohmethanol, welches außer Methanol als Solchem auch Wasser und weitere kondensierbare Nebenprodukt enthalten kann. Der Wassergehalt des Rohmethanols steigt dabei mit der Konzentration von Kohlendioxid im Einsatzgas.

Methanol wird durch Kühlung und dadurch erfolgende Kondensation vom verbleibenden Synthesegasstrom abgetrennt, womit überwiegend nicht reagiertes Synthesegas in der Gasphase verbleibt. Dieses wird in die jeweils folgende der in Serie angeordneten Reaktorstufen eingeschleust. Wie für den Fachmann offensichtlich bildet dabei die letzte der in Serie angeordneten Reaktorstufen eine Ausnahme. Aus der letzten der in Serie angeordneten Reaktorstufen abgezogenes nicht reagiertes Synthesegas wird als Rückführgasstrom in die zweite Kompressorstufe eingeschleust.

Optional wird ein bestimmter Anteil des Rückführgasstroms als Purge-Gasstrom vom Rückführgasstrom abgetrennt. Nicht reagiertes Synthesegas wird in diesem Fall zumindest teilweise aus zumindest einer der Reaktorstufen als Rückführgasstrom zum Einschleusen des Rückführgasstroms in die zweite Kompressorstufe gemäß Schritt c) abgezogen.

Gemäß Schritt e) wird Methanol durch Kühlung und Kondensation aus dem erhaltenen Produktstrom zumindest teilweise von nicht reagiertem Synthesegas abgetrennt. Dabei wird eine vollständige Abtrennung von Methanol durch Kühlung und Kondensation von nicht reagiertem Synthesegas angestrebt, um das Reaktionsgleichgewicht auf die Seite des Produkts (Methanol) zu verschieben.

Eine bevorzugte Ausführungsform des erfindungsgemäßen Verfahrens ist dadurch gekennzeichnet, dass der Anteil an Kohlenmonoxid (CO) im Einsatzgas, in Bezug auf die Gesamtmenge der Kohlenstoffoxide, mehr als 20 Vol.-% beträgt, bevorzugt mehr als 50 Vol.-% beträgt, besonders bevorzugt mehr als 75 Vol.-% beträgt und weiter bevorzugt mehr als 90 Vol.-% beträgt.

Eine bevorzugte Ausführungsform des erfindungsgemäßen Verfahrens ist dadurch gekennzeichnet, dass der Rückführgasstrom nach dem Abziehen gemäß Schritt f) ohne vorhergehende Verdichtung in die zweite Kompressorstufe eingeschleust wird.

Vorzugsweise wird der Rückführgasstrom unmittelbar und ohne den Einsatz eines Rückführgas-Kompressors (Recyclegas-Kompressor), das heißt ohne den Einsatz einer weiteren Kompressorstufe zur Verdichtung des Rückführgases, der zweiten Kompressorstufe zugeführt. Dadurch sinkt nicht nur die gesamte erforderliche Kompressorleistung des Verfahrens (Rückgang des OPEX). Es kann zusätzlich auch auf die Anschaffung eines Rückführgas-Kompressors verzichtet werden, wodurch auch die Investitionskosten (CAPEX) für das Verfahren sinken. Für die gesamte Methanolsynthese sind in diesem Fall lediglich zwei Kompressorstufen erforderlich, nämlich die erste Kompressorstufe zur Verdichtung des ersten Frischgases zur Erzeugung des zweiten Frischgases, sowie die zweite Kompressorstufe zur Verdichtung des zweiten Frischgases zusammen mit dem Rückführgas auf Synthesedruck, zur Erzeugung des Synthesegases.

Eine bevorzugte Ausführungsform des erfindungsgemäßen Verfahrens ist dadurch gekennzeichnet, dass gemäß Schritt e) nicht reagiertes Synthesegas vollständig in die jeweils folgende der in Serie angeordneten Reaktorstufen eingeschleust wird.

Dabei wird die maximal mögliche Menge an nicht reagiertem Synthesegas in die jeweils folgende der in Serie angeordneten Reaktorstufen eingeschleust, was hier unter "vollständig" zu verstehen ist. Wie dem Fachmann in diesem Fall bekannt, wird stets ein kleiner Teil des nicht reagierten Synthesegases im Kondensat (Methanol) gelöst. Dieser kleine Teil kann dementsprechend nicht in die folgende Reaktorstufe eingeschleust werden.

Gemäß dieser Ausführungsform fällt nicht reagiertes Synthesegas nur in der letzten der in Serie angeordneten Reaktorstufen an und die Menge des Rückführgasstroms wird auf ein Minimum reduziert.

Eine bevorzugte Ausführungsform des erfindungsgemäßen Verfahrens ist somit ferner dadurch gekennzeichnet, dass gemäß Schritt f) nicht reagiertes Synthesegas aus der letzten der Mehrzahl in Serie angeordneten Reaktorstufen als Rückführgasstrom abgezogen wird, insbesondere ausschließlich aus der letzten der Mehrzahl in Serie angeordneten Reaktorstufen abgezogen wird.

Eine bevorzugte Ausführungsform des erfindungsgemäßen Verfahrens ist dadurch gekennzeichnet, dass der Rückführgasstrom und der zweite Frischgasstrom zusammengeführt und als kombinierter Gasstrom in die zweite Kompressorstufe eingeschleust werden.

Beide Ströme werden in diesem Fall nach der Zusammenführung zunächst gemischt und anschließend als kombinierter Gasstrom in die zweite Kompressorstufe eingeschleust.

Eine bevorzugte Ausführungsform des erfindungsgemäßen Verfahrens ist dadurch gekennzeichnet, dass das Einsatzgas eine Stöchiometriezahl SN, wobei gilt

$$SN = \frac{n(H_2) - n(CO_2)}{n(CO) + n(CO_2)}, mit\ n\ in\ [mol],$$

von 1,5 bis 3,0 aufweist.

Die Stöchiometriezahl gibt die stöchiometrischen Verhältnisse der Methanolsynthesereaktion

$$CO + 2\ H_2 \rightleftharpoons CH_3OH$$

$$CO_2 + 3\ H_2 \rightleftharpoons CH_3OH + H_2O$$

wieder. Bei idealen stöchiometrischen Verhältnissen und der Abwesenheit von Kohlendioxid liegt die Stöchiometriezahl bei 2. Untersuchungen haben jedoch gezeigt, dass kleinere Kohlendioxid-Mengen den CO-Umsatz im Vorreaktor erhöhen können, so dass die Stöchiometriezahl vorzugsweise in einem Bereich von 1,8 bis 3,0 variiert wird. Bevorzugt gilt dabei 1,8 ≤ SN ≤ 2,5 und besonders bevorzugt 1,9 ≤ SN ≤ 2,2 und weiter bevorzugt 1,95 ≤ SN ≤ 2,05.

Eine bevorzugte Ausführungsform des erfindungsgemäßen Verfahrens ist dadurch gekennzeichnet, dass für eine Rezirkulationsrate R, definiert als

$$R = \frac{Volumenstrom\ (Rückführgasstrom)}{Volumenstrom\ (zweiter\ Frischgasstrom)},$$

0,15 ≤ R ≤ 1,25 gilt.

Dabei werden unter den Volumenströmen jeweils die genormten Volumenströme, bezogen auf den physikalischen Normzustand des jeweiligen Gases bei 0 °C und 1,01325 bar Absolutdruck (Normvolumenstrom), verstanden.

Im Vergleich zu bekannten Verfahren sind die Rezirkulationsraten gemäß erfindungsgemäßem Verfahren durch die Verwendung einer Mehrzahl in Serie angeordneter Reaktionsstufen mit Zwischenkondensation sehr niedrig.

Dabei ist weiter bevorzugt, dass für die Rezirkulationsrate R gilt: 0,1 ≤ R ≤ 0,5.

Insbesondere bei der Verwendung unterstöchiometrischer Einsatzgase, also Einsatzgasen mit einer Stöchiometriezahl von kleiner als 2, kann die Rezirkulationsrate bei konstant hohem Wasserstoffumsatz weiter reduziert werden, wie Untersuchungen überraschenderweise gezeigt haben. Wird die Menge des Rückführgasstroms und somit die Rezirkulationsrate weiter reduziert, insbesondere auf Werte deutlich kleiner als 1, sinkt entsprechend auch die gesamte erforderliche Kompressorleistung.

Eine bevorzugte Ausführungsform des erfindungsgemäßen Verfahrens ist dadurch gekennzeichnet, dass über die Gesamtheit der Mehrzahl der in Serie angeordneten Reaktorstufen, ausgehend vom Einsatzgas, ein Kohlenstoffoxide-Umsatz von wenigstens 80 mol-% erzielt wird, bevorzugt von mindestens 90 mol-% erzielt wird, besonders bevorzugt von mindestens 95 mol-% erzielt wird.

Mit steigendem Kohlenstoffoxide-Umsatz verringert sich die Menge des nicht reagierten Synthesegases und damit auch die Menge an Rückführgas. Entsprechend höher fallen die Einsparungen bezüglich der Kompressorleistung mit steigendem Umsatz aus. Der Umsatz über die Gesamtheit der in Serie angeordneten Reaktorstufen bei einmaligem Durchgang des Synthesegases wird auch als "per-pass" Umsatz bezeichnet. Der per-pass Umsatz steigt mit der Anzahl der Reaktorstufen und Zwischenkondensationsschritte. Unter "Kohlenstoffoxide-Umsatz", auch als "COx-Umsatz" bezeichnet wird der gemeinsame Umsatz von Kohlenmonoxid (CO) und Kohlendioxid ($CO_2$) zu Methanol verstanden.

Eine bevorzugte Ausführungsform des erfindungsgemäßen Verfahrens ist dadurch gekennzeichnet, dass die Mehrzahl der in Serie angeordneten Reaktorstufen eine Anzahl von 2 bis 8 Reaktorstufen umfasst.

Die optimale Anzahl der Reaktorstufen hängt von einer Vielzahl von Faktoren wie der zu produzierenden Methanol-Menge, des Umsatzes pro Reaktorstufen, Zusammensetzung des Einsatzgases sowie gegebenenfalls weiterer Faktoren ab. Allgemein steigen die Investitionskosten mit der Anzahl der Reaktorstufen an und die Menge des Rückführgasstroms nimmt ab, wodurch Einsparungen bezüglich der Kompressorleistung besonders hoch sind. Umgekehrt sinken die Investitionskosten mit der Verringerung der Anzahl an Reaktionsstufen, jedoch steigt die Menge des Rückführgasstrom an, wodurch die Einsparungen bezüglich der Kompressorleistung geringer ausfallen.

Vorzugsweise umfasst die Mehrzahl der in Serie angeordneten Reaktorstufen daher eine Anzahl von 3 bis 5 Reaktorstufen, besonders bevorzugt eine Anzahl von 4 Reaktorstufen. Dadurch wird ein optimaler Kompromiss im Hinblick auf Investitionskosten und Einsparung von Kompressorleistung erzielt.

Die Aufgaben der Erfindung werden ferner zumindest teilweise gelöst durch eine Anlage für die Herstellung von Methanol, aufweisend folgende miteinander in Fluidverbindung stehende Anlagenelemente:

Eine erste Kompressorstufe zum Vorverdichten eines Kohlenstoffoxide und Wasserstoff ($H_2$) aufweisenden ersten Frischgases zur Erzeugung eines zweiten Frischgases;

eine stromabwärts zur ersten Kompressorstufe angeordnete zweite Kompressorstufe zur Verdichtung des zweiten Frischgases und eines Rückführgases zu einem Synthesegas bei Synthesedruck;

eine stromabwärts zur zweiten Kompressorstufe angeordnete Mehrzahl in Serie angeordneter Reaktoreinheiten zur Erzeugung eines Methanol und nicht reagiertes Synthesegas aufweisenden Produktstroms aus Synthesegas, wobei jede der Reaktoreinheiten

- einen ein Katalysatorbett aufweisenden Reaktor,
- einen Wärmeaustauscher zur Kühlung des Produktstroms, sowie
- einen Abscheider zur Abtrennung von Methanol von nicht reagiertem Synthesegas

umfasst, wobei
jede der Reaktoreinheiten einen Auslass zum Abziehen von Methanol umfasst und jede der Reaktoreinheiten einen Auslass zum Zuführen von nicht reagiertem Synthesegas zu einer jeweils nachfolgend angeordneten Reaktoreinheit und/oder einen Auslass zum Zuführen von nicht reagiertem Synthesegas als Rückführgas zur zweiten Kompressorstufe umfasst, wobei
eine Zuführung zum Einschleusen des Rückführgases in die zweite Kompressorstufe zwischen der ersten und zweiten Kompressorstufe angeordnet ist.

Der erfindungsgemäßen Anlage ist eine Produktionseinheit zur Erzeugung eines Wasserstoff ($H_2$) und Kohlenstoffoxide aufweisenden Einsatzgases vorgeschaltet.
Das Einsatzgas weist in Bezug auf die Gesamtmenge der Kohlenstoffoxide bevorzugt einen Anteil von mehr als 20 Vol.-% Kohlenmonoxid (CO) auf, besonders bevorzugt einen Anteil von mehr als 50 Vol.-% , oder mehr als 75 Vol.-%, oder mehr als 90 Vol.-%.
Das Einsatzgas wird der erfindungsgemäßen Anlage als erstes Frischgas zugeführt. Eine bevorzugte Ausführungsform der erfindungsgemäßen Anlage ist dadurch gekennzeichnet, dass zwischen dem Auslass zum Zuführen von nicht reagiertem Synthesegas als Rückführgas zur zweiten Kompressorstufe und der Zuführung zum Einschleusen des Rückführgases in die zweite Kompressorstufe keine Kompressorstufe zur vorherigen Verdichtung des Rückführgases vor dem Einschleusen in die zweite Kompressorstufe angeordnet ist.
Vorzugsweise wird das nicht reagierte Synthesegas nach Abziehen aus einer Reaktoreinheit als Rückführgas unmittelbar der zweiten Kompressorstufe zugeführt. In diesem Fall ist zwischen dem Auslass der Reaktoreinheit und der Zuführung zur zweiten Kompressoreinheit keine zusätzliche Kompressorstufe zur Verdichtung des Rückführgases vor dem Zuführen zur zweiten Kompressorstufe vorgesehen.
Eine bevorzugte Ausführungsform der erfindungsgemäßen Anlage ist dadurch gekennzeichnet, dass ein Auslass zum Zuführen von nicht reagiertem Synthesegas als Rückführgas zur zweiten Kompressorstufe an der letzten der Mehrzahl in Serie angeordneten Reaktoreinheiten angeordnet ist, vorzugsweise ausschließlich an der letzten der Mehrzahl in Serie angeordneten Reaktoreinheiten angeordnet ist.
Vorzugsweise weisen alle Reaktoreinheiten, mit Ausnahme der letzten der in Serie angeordneten Reaktoreinheiten, einen Auslass zum Zuführen von nicht reagiertem Synthesegas einer Reaktoreinheit zu einer jeweils nachfolgend angeordneten Reaktoreinheit auf. Nur die letzte der in Serie angeordneten Reaktoreinheiten weist einen Auslass zum Zuführen von nicht reagiertem Synthesegas einer Reaktoreinheit als Rückführgas zur zweiten Kompressorstufe auf. Dadurch wird die Menge des Rückführgases auf ein Minimum reduziert.
Die Aufgaben der Erfindung werden ferner zumindest teilweise gelöst durch die Verwendung der erfindungsgemäßen Anlage in einem Verfahren gemäß der Erfindung.

## Einsatzgas

Das Einsatzgas weist zumindest Wasserstoff ($H_2$) und Kohlenstoffoxide auf. Unter dem Begriff "Kohlenstoffoxide" werden die Verbindungen Kohlenmonoxid (CO) sowie Kohlendioxid ($CO_2$) subsummiert. In Bezug auf die Gesamtmenge der Kohlenstoffoxide weist das Einsatzgas vorzugsweise einen Kohlenmonoxid-Anteil von wenigstens 20 Vol.-% auf. Vorzugsweise ist das Einsatzgas hochkohlenmonoxidhaltig. In einem Beispiel weist das Einsatzgas in Bezug auf die Kohlenstoffoxide wenigstens 50 Vol.-% Kohlenmonoxid auf, oder zumindest 70 Vol.-%, oder zumindest 90 Vol.-%, oder zumindest 95 Vol.-%, oder zumindest 99 Vol.-%. In einem Beispiel weist das Einsatzgas in Bezug auf die Kohlenstoffoxide nahezu ausschließlich Kohlenmonoxid auf, wobei Kohlendioxid in diesem Fall nur noch in Spuren vorhanden ist. Ein solches Einsatzgas kann beispielsweise durch Behandlung eines Roh-Einsatzgases in einer Methanol-Wäsche erhalten werden. Im Rahmen einer Methanol-Wäsche oder anderer geeigneter Gaswäscheverfahren kann Kohlendioxid praktisch vollständig entfernt werden. Ein besonders hierfür geeignetes Verfahren ist das selektive Rectisol®-Verfahren.
Das Einsatzgas kann aus jeder dem Fachmann bekannten Quelle stammen. In einem Beispiel stammt das Einsatzgas aus einem Reformierungsverfahren. Dabei wird das Wasserstoff und Kohlenstoffoxide aufweisende Einsatzgas durch Reformierung eines Kohlenwasserstoffe aufweisenden Rohmaterials wie Methan oder Erdgas erzeugt. Der Reformierungsprozess kann einen oder mehrere Schritte wie Dampfreformierung, partielle Oxidation oder autotherme Reformierung aufweisen. Optional wird das Reformierungsprodukt in einer Wassergas-Shift-Reaktion weiter umgesetzt, um den Wasserstoff-Gehalt zu erhöhen, wobei gleichzeitig der Kohlendioxid-Anteil erhöht wird. Durch eine Wassergas-Shift kann beispielsweise die Stöchiometriezahl eines Einsatzgases durch Mischung mit einem nicht geshifteten Synthesegas gezielt eingestellt werden.
Das Einsatzgas kann, unabhängig von der Quelle aus der es stammt, bei einer Temperatur zwischen 400 °C und 1200 °C und/oder bei einem Druck zwischen 10 und 60 bar erzeugt werden. Das Einsatzgas kann außer den vorgenannten Bestandteilen auch unterschiedliche Mengen inerter Bestandteile wie Methan oder Stickstoff enthalten. Unter inerten Bestanteilen sind dabei insbesondere unter den Bedingungen der Methanolsynthese inerte Bestandteile

zu verstehen, das heißt Bestandteile welche unter den Bedingungen der Methanolsynthese nicht zu Methanol oder (unerwünschten) Nebenprodukten umgesetzt werden.

Das Einsatzgas wird üblicherweise unter den Taupunkt von Dampf zur Auskondensation von Wasser abgekühlt, bevor es als erstes Frischgas im erfindungsgemäßen Verfahren verwendet wird. Insbesondere wird das Einsatzgas auf unter 100 °C, vorzugsweise auf unter 60 °C, und weiter bevorzugt auf 40 °C oder weniger abgekühlt, um Wasser nach Kondensation vom Einsatzgas abzutrennen. Das erste Frischgas ist somit insbesondere frei oder weitgehend frei von Wasser.

Methanol

Ist in Zusammenhang mit Gegenständen der Erfindung von "Methanol" die Rede, so ist damit in der Regel ein Rohmethanol gemeint, welches außer Methanol als Solchem auch Wasser und weitere kondensierbare Nebenprodukte enthalten kann. Dieses Rohmethanol wird in dem erfindungsgemäßen Verfahren oder der erfindungsgemäßen Anlage nachgeschalteten Verfahren oder Anlagen einer Aufreinigung zur Gewinnung von Reinmethanol zugeführt.

Kompressorstufe

Ist in Zusammenhang mit Gegenständen der Erfindung von einer "Kompressorstufe" die Rede, so ist darunter entweder ein Verfahrensschritt zu verstehen, bei dem eine Gasmenge von einem Ausgangsdruck $p_1$ auf einen Enddruck $p_2$ in einem Schritt verdichtet wird (mit $p_2 > p_1$) oder eine apparative Einheit, die zur Verdichtung einer Gasmenge von einem Ausgangsdruck $p_1$ auf einen Enddruck $p_2$ (mit $p_2 > p_1$) in einem Schritt geeignet ist. Der Quotient aus $p_2$ und $p_1$ beschreibt dabei das Kompressionsverhältnis der jeweiligen Kompressorstufe.

Ein "Kompressor" oder eine "Kompressoreinheit" kann dabei mehrere Kompressorstufen umfassen, wobei die Verdichtung von $p_1$ auf $p_2$ dann in mehreren Schritten erfolgt, wobei jeder der Schritte ein definiertes Kompressionsverhältnis aufweist.

In einem Beispiel können mehrere Kompressorstufen in einen Kompressor integriert sein, wobei dieser Kompressor vorzugsweise eine Zwischenkühlung nach der ersten und/oder zweiten Kompressorstufe aufweist. In einem weiteren Beispiel umfassen zwei hintereinandergeschaltete Kompressoren jeweils nur eine Kompressorstufe.

Erste Kompressorstufe, erstes Frischgas

Das erste Frischgas wird der ersten Kompressorstufe zugeführt. In der ersten Kompressorstufe findet eine Verdichtung des ersten Frischgases statt, wobei ein zweites Frischgas erhalten wird. Das zweite Frischgas weist gegenüber dem ersten Frischgas einen höheren Druck auf.

In einem Beispiel weist das erste Frischgas einen Druck auf, der zumindest weitgehend dem Druck des Einsatzgases entspricht, beispielsweise einen Druck von 10 bis 60 bar, vorzugsweise einen Druck von 25 bis 45 bar und weiter bevorzugt einen Druck von 20 bis 40 bar.

Zweite Kompressorstufe, zweites Frischgas

Das zweite Frischgas weist in einem Beispiel einen Druck von 50 bis 70 bar auf, und vorzugsweise einen Druck von 60 bis 70 bar.

Das zweite Frischgas wird, zusammen mit dem Rückführgas, der zweiten Kompressorstufe zugeführt und verdichtet, wobei das für die katalytische Umsetzung zu Methanol erforderliche Synthesegas erhalten wird. Das Synthesegas weist gegenüber dem zweiten Frischgas und/oder dem Rückführgas einen höheren Druck auf.

In einem Beispiel weist das Synthesegas einen Druck von 60 bis 120 bar, vorzugsweise einen Druck von 70 bis 100 bar und weiter bevorzugt einen Druck von 80 bis 90 bar.

Synthesedruck

In der zweiten Kompressorstufe werden das zweite Frischgas und das Rückführgas insbesondere als kombinierter Gasstrom auf Synthesedruck verdichtet. Synthesedruck ist der für die Methanolsynthese erforderliche Druck, den das Synthesegas beispielsweise beim Einschleusen in die erste der Mehrzahl von Reaktorstufen aufweisen sollte. In einem Beispiel entspricht der Synthesedruck im Wesentlichen dem Druck des Synthesegases von beispielsweise 60 bis 120 bar, vorzugweise von 70 bis 100 bar und weiter bevorzugt von 80 bis 90 bar.

Katalytische Umsetzung

Das Synthesegas wird in den Reaktorstufen katalytisch zu Methanol umgesetzt

Die Temperatur des Synthesekatalysators beläuft sich in geeigneter Weise auf 180 bis 300 °C, vorzugsweise mit einer Spitzentemperatur von nicht mehr als 280 °C. Das Synthesegas tritt in eine Reaktorstufe vorzugsweise mit einer Temperatur von 200 °C bis 250 °C ein und tritt aus einer Reaktorstufe vorzugsweise mit einer Temperatur von 220 °C bis 270 °C wieder aus.

Der für die Methanolsynthese verwendete Festbett-Katalysator ist vorzugsweise ein kupferbasierter Katalysator. Insbesondere geeignet sind hierbei kupferbasierte Katalysatoren welche Verbindungen wie Zinkoxid, Aluminiumoxid, Chromoxid, Titanoxid, Zirkoniumoxid (Zirkon) und/oder Magnesiumoxid aufweisen.

Geeignete Reaktortypen sind wassergekühlte Reaktoren, welche siedendes Kesselspeisewasser zur Kühlung verwenden oder gasgekühlte Reaktoren, in denen die Kühlung durch nicht umgesetztes Synthesegas erfolgt, welches dabei erwärmt und vorgeheizt in die nächste Reaktorstufe eingeschleust werden kann.

Reaktorstufe, Reaktoreinheit

Grundsätzlich wird im Zusammenhang mit Gegenständen der Erfindung unter einer "Reaktorstufe" ein Verfahrensschritt und/oder eine Vorrichtung verstanden, welcher/welche zur Durchführung einer chemischen Reaktion geeignet ist. Unter einer "Reaktoreinheit" wird in Zusammenhang mit Gegenständen der Erfindung eine apparative Vorrichtung verstanden, welche zur Durchführung einer chemischen Reaktion geeignet ist.

Eine Reaktorstufe oder eine Reaktoreinheit kann außer dem eigentlichen Reaktor insbesondere einen dem Reaktor nachgeschalteten Wärmeaustauscher zum Kühlen des Produktstroms, insbesondere zur Kondensation von Methanol, umfassen. Ferner umfasst eine Reaktorstufe oder eine Reaktoreinheit insbesondere einen Abscheider, beispielsweise zur Abtrennung von kondensiertem Methanol von nicht umgesetztem Synthesegas. Auslässe einer Reaktorstufe oder einer Reaktoreinheit zum Abziehen von nicht reagiertem Synthesegas, welches in weiteren Schritten der folgenden Reaktorstufe oder Reaktoreinheit zugeführt oder der zweiten Kompressorstufe als Rückführgas zugeführt wird, befinden sich insbesondere am Abscheider-Teil der Reaktorstufe oder Reaktoreinheit.

Der Produktstrom wird durch Wärmeaustauscher vorzugsweise auf eine Temperatur von kleiner als 50 °C gekühlt, um Methanol zu kondensieren und im Abscheider von nicht reagiertem Synthesegas zu trennen.

Produktstrom

Durch die katalytische Umsetzung des Synthesegases an einem Festbett-Katalysator bei erhöhter Temperatur wird ein Produktstrom erhalten, der Methanol (Rohmethanol, aufweisend Methanol, Wasser und kondensierbare Nebenprodukte) und nicht reagiertes Synthesegas umfasst. Insbesondere bei Verwendung von hochkohlendioxidhaltigen Einsatzgasen wird Wasser zwangsläufig als signifikanter Bestandteil im Rohmethanol erhalten. Da Wasserdampf insbesondere bei hohen Partialdrücken zu einer sukzessiven Deaktivierung des Synthesekatalysators führt, wird dieses zusammen mit Methanol als Solchem sowie kondensierbaren Nebenprodukten durch Kühlung und Abscheidung von nicht reagiertem Synthesegas getrennt. Kondensierbare Nebenprodukte sind beispielsweise Dimethylether, Methylformiat, Aceton, Ethanol sowie höhere Alkohole.

Nicht reagiertes Synthesegas

Der jeweiligen Reaktorstufe zugeführtes Synthesegas wird in Abhängigkeit von der thermodynamischen Gleichgewichtskonstante, welche ein Funktion der Temperatur ist, stets nur teilweise zu Methanol umgesetzt. Nicht umgesetztes Synthesegas ist nicht reagiertes Synthesegas. Dieses wird entweder einer folgenden der Mehrzahl in Serie angeordneten Reaktorstufen zugeführt oder als Rückführgas der zweiten Kompressorstufe zugeführt.

Rückführgas

Derjenige Anteil des nicht reagierten Synthesegases, der nach Austritt aus einer Reaktorstufe nicht einer folgenden der Mehrzahl von Reaktorstufen zugeführt wird, wird insbesondere als Rückführgas zur zweiten Kompressorstufe zurückgeführt.

Dabei kann ein Teil des Rückführgasstroms als Spülstrom, auch als Purgestrom bezeichnet, vom Rückführgasstrom abgezweigt werden. Bei der Methanolsynthese werden am Katalysatorbett einer Reaktorstufe Wasserstoff sowie Kohlenstoffoxide verbraucht. Unter den Bedingungen der Methanolsynthese inerte Bestandteile wie Stickstoff, Argon oder Methan werden nicht verbraucht und können sich über einen längeren Zeitraum im Synthesekreislauf anreichern. Die Anreicherung inerter Gasbestandteile ist nicht erwünscht, weswegen insbesondere ein Teil des Rückführgasstroms als Spülstrom aus dem Synthesekreislauf abgezweigt wird. Der Synthesekreislauf wird zumindest durch die zweite Kompressorstufe, die Mehrzahl der Reaktorstufen sowie den Gasströmen zwischen diesen Elementen gebildet.

Üblicherweise wird aus dem Spülstrom Wasserstoff zurückgewonnen, beispielsweise durch eine Wechseldruckabsorptionsvorrichtung oder mit Hilfe eines Membransystems. Aus dem Spülstrom zurückgewonnener Wasserstoff kann beispielsweise dem ersten Frischgasstrom zugeführt werden.

Vorzugsweise wird das Rückführgas nicht verdichtet, nachdem es aus zumindest einer der Reaktorstufen abgezogen wurde und bevor es der zweiten Kompressorstufe zugeführt wird. Das Rückführgas weist in einem Beispiel einen Druck von 60 bis 80 bar auf, vorzugsweise einen Druck von 65 bar bis 75 bar.

## Ausführungsbeispiele

Die Erfindung wird im Folgenden durch Vergleichsbeispiele und ein erfindungsgemä-ßes Beispiel näher erläutert, ohne den Gegenstand der Erfindung dadurch zu beschränken. Weitere Merkmale, Vorteile und Anwendungsmöglichkeiten der Erfindung ergeben sich aus der nachfolgenden Beschreibung der Beispiele in Zusammenhang mit den Zeichnungen und den Zahlenbeispielen.

Es zeigt

Figur 1 ein schematisches Blockfließbild eines Herstellverfahrens für Methanol mit Rückführgaskompressorstufe im Synthesekreislauf gemäß Stand der Technik,

Figur 2 ein schematisches Blockfließbild eines Herstellverfahrens für Methanol mit Rückführgaskompressorstufe im Synthesekreislauf gemäß Stand der Technik,

Figur 3 ein schematisches Blockfließbild eines erfindungsgemäßen Herstellverfahrens für Methanol mit einer Mehrzahl in Serie angeordneter Reaktorstufen ohne Rückführgaskompressorstufe im Synthesekreislauf.

Figur 1 zeigt ein stark vereinfachtes schematisches Blockfließbild eines Herstellverfahrens für Methanol mit einer Rückführgaskompressorstufe in einem Synthesekreislauf mit einer einzigen Reaktorstufe und einer einzigen Kompressorstufe für Frischgas.

Einer ersten Kompressorstufe K1 wird ein erster Frischgasstrom 101 mit einem Druck von 34 bar zugeführt. Das Frischgas weist einen Wasserstoffanteil von 68,9 Vol.-% auf, einen Kohlenmonoxid-Anteil von 18,9 Vol.-% und einen Kohlendioxid-Anteil von 10,5 Vol.-%. Aufgrund der molaren Verhältnisse der Frischgas-Komponenten ergibt sich für das Frischgas des Frischgasstroms 101 eine Stöchiometriezahl SN von 2,00. Der Frischgasstrom 101 wird durch die erste Kompressorstufe K1 verdichtet, so dass auf der Druckseite der Kompressorstufe K1 ein zweiter Frischgasstrom 102 resultiert. Der zweite Frischgasstrom 102 wird mit dem Rückführgasstrom 104b zusammengeführt, wodurch ein kombinierter Gasstrom resultiert, der hier als Synthesegasstrom 103 bezeichnet wird. Die Verdichtung durch Kompressorstufe K1 bewirkt, dass der Synthesegasstrom 102 am Eintritt der Reaktorstufe R1 einen Druck von 85,0 bar aufweist. Synthesegas des Synthesegasstroms 102 wird in Reaktorstufe R1 an einem Kupfer basierten Festbettkatalysator mit einem Katalysatorvolumen von 115 $m^3$ zu Methanol umgesetzt. Der Druck am Reaktoraustritt des Reaktors von R1 beträgt 78,0 bar. Die Umsetzung ist unvollständig, wodurch ein Strom nicht reagierten Synthesegases 105 erhalten wird, der teilweise als Rückführgasstrom 104a zur Rückführgaskompressorstufe RK geführt und verdichtet wird, so dass Rückführgasstrom 104b auf der Druckseite der Rückführgaskompressorstufe RK erhalten wird. Ein Teil des nicht reagierten Synthesegases 105 wird als Purgestrom 107 aus dem Synthesekreislauf entfernt, um die Akkumulation von inerten Bestandteilen und von Nebenprodukten der Methanolsynthese zu vermeiden. Reaktorstufe R1 weist außer dem eigentlichen Reaktor auch einen Wärmeaustauscher zur Kühlung des Produktstroms sowie einen Abscheider auf. Durch Kühlung des Produktstroms und Kondensation von Methanol im Abscheider wird ein Methanol aufweisender Produktstrom 106 erhalten. Das Methanol liegt als Rohmethanol vor und wird zur Gewinnung von Reinmethanol einer in Abhängigkeit von der erforderlichen Reinheit geeigneten Aufarbeitung zugeführt (nicht gezeigt).

Der Kohlenstoffoxide-Umsatz (COx-Umsatz) per-pass, das heißt pro Reaktordurchgang, beträgt im Vergleichsbeispiel gemäß Figur 1 54,5 %. Der geringe per-pass Umsatz erfordert die Rückführung und Rückverdichtung einer größeren Menge nicht reagierten Synthesegases 105 als Rückführgas 104a.

Die Rezirkulationsrate R, das heißt das Verhältnis der Menge an Rückführgas 104a oder 104b zu Frischgas 102, beträgt im Vergleichsbeispiel der Figur 1 R = 2,4. Somit entspricht die von der Rückführgaskompressorstufe zu verdichtende Rückführgasmenge in etwa dem zweieinhalbfachen der Frischgasmenge, jeweils bezogen auf die Normvolumenströme von Rückführgas und Frischgas. Dadurch wird trotz des geringen per-pass Umsatzes eine Kohlenstoff-Effizienz von 99,1 % erzielt, was bedeutet dass 99,1 % des durch das Frischgas eingesetzten Kohlenstoffs zu Methanol umgesetzt wird. Bei einer Produktion von 2410 Tonnen Rohmethanol pro Tag (t/d) ist gemäß Vergleichsbeispiel der Figur 1 eine Kompressorleistung von 12,5 MW für K1 und 2,4 MW für RK erforderlich, was einer Gesamtkompressorleistung von 14,9 MW entspricht.

Figur 2 zeigt ein stark vereinfachtes schematisches Blockfließbild eines Herstellverfahrens für Methanol mit einer Rückführgaskompressorstufe in einem Synthesekreislauf mit einer Reaktorstufe und zwei in Serie geschalteten Kompressorstufen für Frischgas. Das Verfahren der Figur 2 unterscheidet sich von dem Verfahren der Figur 1 somit in der Verwendung einer zusätzlichen Kompressorstufe für Frischgas.

Einer ersten Kompressorstufe K1 wird ein erster Frischgasstrom 201 mit einem Druck von 34 bar zugeführt. Das Frischgas weist einen Wasserstoffanteil von 68,9 Vol.-% auf, einen Kohlenmonoxid-Anteil von 18,9 Vol.-% und einen Kohlendioxid-Anteil von 10,5 Vol.-%. Aufgrund der molaren Verhältnisse der Frischgas-Komponenten ergibt sich für das Frischgas des Frischgasstroms 101 eine Stöchiometriezahl SN von 2,00. Der Frischgasstrom 201 wird durch die erste Kompressorstufe K1 verdichtet, so dass auf der Druckseite der Kompressorstufe K1 ein zweiter Frischgasstrom 202 mit einem Druck von 60,0 bar erhalten wird. Der zweite Frischgasstrom 202 wird durch die zweite Kompressorstufe K2 verdichtet, wobei ein dritter Frischgasstrom 203 auf der Druckseite von K2 erhalten wird. Der dritte Frischgasstrom 203 wird mit Rückführgasstrom 206b zusammengeführt, wodurch ein kombinierter Gasstrom resultiert, der hier als Synthesegasstrom 204 bezeichnet wird. Die Verdichtung durch Kompressorstufe K2 bewirkt, dass der Synthesegasstrom 204 am Eintritt der Reaktorstufe R1 einen Druck von 85,0 bar aufweist. Synthesegas des Synthesegasstroms 204 wird in Reaktorstufe R1 an einem Kupfer basierten Festbettkatalysator mit einem Katalysatorvolumen von 115 $m^3$ zu Methanol umgesetzt. Der Druck am Reaktoraustritt beträgt 78,0 bar. Die Umsetzung ist unvollständig, wodurch ein Strom nicht reagierten Synthesegases 205 erhalten wird, der teilweise als Rückführgasstrom 206a zur Rückführgaskompressorstufe RK geführt und verdichtet wird, so dass Rückführgasstrom 206b auf der Druckseite der Rückführgaskompressorstufe RK erhalten wird. Ein Teil des nicht reagierten Synthesegases 205 wird als Purgestrom 208 aus dem Kreislauf entfernt, um die Akkumulation von inerten Bestandteilen zu vermeiden. Reaktorstufe R1 weist außer dem eigentlichen Reaktor auch einen Wärmeaustauscher zur Kühlung des Produktstroms sowie einen Abscheider auf. Durch Kühlung des Produktstroms und Kondensation von Methanol im Abscheider wird ein Methanol aufweisender Produktstrom 207 erhalten. Das Methanol liegt als Rohmethanol vor und wird in Abhängigkeit von der geforderten Reinheit einer geeigneten Aufarbeitung zur Gewinnung von Reinmethanol zugeführt (nicht gezeigt).

Der Kohlenstoffoxide-Umsatz (COx-Umsatz) per-pass, das heißt pro Reaktordurchgang, beträgt im Vergleichsbeispiel gemäß Figur 2 54,5 %. Der geringe per-pass Umsatz erfordert die Rückführung und Rückverdichtung einer größeren Menge nicht reagierten Synthesegases 205 als Rückführgas 206a.

Die Rezirkulationsrate R, das heißt das Verhältnis der Menge an Rückführgas 206a oder 206b zu Frischgas 203 beträgt im Vergleichsbeispiel der Figur 2 ebenfalls R = 2,4. Somit entspricht die von der Rückführgaskompressorstufe zu verdichtende Rückführgasmenge in etwa dem zweieinhalbfachen der Frischgasmenge, jeweils bezogen auf die Normvolumenströme von Rückführgas und Frischgas. Dadurch wird trotz des geringen per-pass Umsatzes eine Kohlenstoff-Effizienz von 99,1 % erzielt, was bedeutet dass 99,1 % des durch das Frischgas eingesetzten Kohlenstoffs zu Methanol umgesetzt wird. Bei einer Produktion von 2410 Tonnen Rohmethanol pro Tag (t/d) ist im Vergleichsbeispiel der Figur 2 eine Kompressorleistung von 11,3 MW für K1 und K2 zusammen sowie von 2,4 MW für RK erforderlich, was einer Gesamtkompressorleistung von 13,7 MW entspricht. Die Gesamtkompressorleistung ist damit niedriger als im Vergleichsbeispiel der Figur 1, jedoch werden drei Kompressorstufen K1, K2 sowie RK benötigt, was den CAPEX der Anlage erhöht.

Figur 3 zeigt ein stark vereinfachtes schematisches Blockfließbild eines erfindungsgemäßen Herstellverfahrens für Methanol ohne Rückführgaskompressorstufe im Synthesekreislauf, wobei der Synthesekreislauf eine Mehrzahl in Serie angeordneter Reaktorstufen umfasst.

Einer ersten Kompressorstufe K1 wird ein erster Frischgasstrom 301 mit einem Druck von 34,0 bar zugeführt. Das Frischgas weist einen Wasserstoffanteil von 68,9 Vol.-% auf, einen Kohlenmonoxid-Anteil von18,9 Vol.-% und einen Kohlendioxid-Anteil von 10,5 Vol.-%. Aufgrund der molaren Verhältnisse der Frischgas-Komponenten ergibt sich für das Frischgas des Frischgasstroms 101 eine Stöchiometriezahl SN von 2,00. Der erste Frischgasstrom 301 wird durch die erste Kompressorstufe K1 verdichtet, so dass auf der Druckseite der Kompressorstufe K1 ein zweiter Frischgasstrom 302 mit einem Druck von 65,0 bar erhalten wird. Der zweite Frischgasstrom 302 wird mit Rückführgasstrom 305 zusammengeführt, wodurch ein kombinierter Gasstrom 303 resultiert, welcher in eine zweite Kompressorstufe K2 eingeschleust und dabei auf einen Druck von 85,0 bar verdichtet wird. Es resultiert auf der Druckseite der zweiten Kompressorstufe ein Gasstrom mit Synthesedruck, der hier als Synthesegasstrom 304 bezeichnet wird.

Der Synthesegasstrom 304 wird in eine erste von insgesamt vier in Serie angeordneten Reaktorstufen R1 bis R4 eingeschleust. In der ersten Reaktorstufe R1 wird Synthesegas 304 an einem Kupfer basierten Festbettkatalysator mit einem Katalysatorvolumen von 28,75 $m^3$ zu Methanol umgesetzt.

Die Reaktorstufen R2, R3 und R4 verfügen jeweils über ein Katalysatorvolumen von 28,75 $m^3$ desselben Kupfer basierten Katalysators, so dass sich ein Gesamtkatalysatorvolumen von 115 $m^3$ ergibt, was dem Katalysatorvolumen der Einzelreaktorstufe in den Vergleichsbeispielen gemäß Figur 1 und Figur 2 entspricht.

Durch die nicht vollständige Umsetzung des Synthesegases 304 in Reaktorstufe R1 wird ein Strom nicht reagierten Synthesegases 306a erhalten, der in die folgende der in Serie angeordneten Reaktorstufen eingeschleust

wird, hier in Reaktorstufe R2. Reaktorstufe R1 und weitere Reaktorstufen R2 bis R4 weisen außer dem eigentlichen Reaktor auch einen Wärmeaustauscher zur Kühlung des Produktstroms sowie einen Abscheider auf. Durch Kühlung des Produktstroms und Kondensation von Methanol im Abscheider wird in R1 ein Produktstrom 307a erhalten.

Analog der katalytischen Umsetzung in R1 wird nicht reagiertes Synthesegas 306a in Reaktorstufe R2 zu Methanol und Nebenprodukten umgesetzt, wodurch ein weiterer Produktstrom 307b erhalten wird. Nicht bei der Umsetzung am Kupfer basierten Katalysator von R2 reagiertes Synthesegas wird wiederum in die folgende Reaktorstufe R3 eingeschleust, wobei ein Produktstrom 307c und nicht reagiertes Synthesegas 306c erhalten werden. Ein kleiner Anteil nicht reagierten Synthesegases R2 wird dabei stets im Kondensat (Methanol) gelöst und steht nicht für die nächste Reaktorstufe zur Verfügung. Nicht reagiertes Synthesegas 306c wird in die letzte der in Serie angeordneten Reaktorstufen R4 eingeschleust, wobei ein weiterer Produktstrom 307d erhalten wird. Die Produktströme 307a bis 307d werden zu einem Gesamtproduktstrom 307 zusammengeführt. Gesamtproduktstrom 307 weist Rohmethanol auf, das in Abhängigkeit von der geforderten Reinheit einer geeigneten Aufarbeitung zur Gewinnung von Reinmethanol zugeführt wird (nicht gezeigt).

Der Druck am Reaktoraustritt der Reaktorstufe R4 beträgt 67,0 bar. Der Druckverlust über alle Reaktorstufen beträgt somit 18,0 bar. In der letzten Reaktorstufe nicht reagiertes Synthesegas 306d wird unmittelbar als Rückführgasstrom 305 zum zweiten Frischgasstrom 302 zurückgeführt und mit diesem zusammengeführt. Ein Teil des in der letzten Reaktorstufe R4 abgezogenen nicht reagierten Synthesegases wird im Beispiel gemäß Figur 3 als Purgegasstrom abgezweigt, um die Akkumulation von inerten Bestandteilen und Nebenprodukten im Rückführgasstrom 305 zu vermeiden.

Der Kohlenstoffoxide-Umsatz (COx-Umsatz) per-pass, das heißt pro Reaktordurchgang über alle Reaktorstufen R1 bis R5, beträgt im erfindungsgemäßen Beispiel gemäß Figur 3 93,9 %. Der hohe per-pass Umsatz erfordert die Rückführung und Rückverdichtung einer vergleichsweise kleinen Menge nicht reagierten Synthesegases 306d als Rückführgas 305.

Die Rezirkulationsrate R, das heißt das Verhältnis der Menge an Rückführgas 305 zu Frischgas 302 beträgt im Beispiel der Figur 3 R = 0,45. Somit entspricht die Rückführgasmenge weniger als der halben Frischgasmenge, jeweils bezogen auf die Normvolumenströme von Rückführgas und Frischgas. Diese geringe Menge erfordert erfindungsgemäß keinen dedizierten Rückführgaskompressor. Vielmehr erfolgt die Rückverdichtung des Rückführgases 305 auf Synthesedruck (85 bar) durch die zweite Kompressorstufe K2. Trotz der geringen Menge an Rückführgas 305 wird aufgrund des hohen per-pass Umsatzes von 93,9 % eine Kohlenstoff-Effizienz von 98,8 % erzielt, was weitgehend der Kohlenstoffeffizienz der Vergleichsbeispiele gemäß Figur 1 und 2 entspricht. Die Rezirkulationsrate ist bei gleicher Kohlenstoffeffizienz um einen Faktor von >5 kleiner als in den Vergleichsbeispielen, was durch den höheren Umsatz und die Zwischenkondensation von Produkten in der Mehrzahl der Reaktorstufen (R1 bis R4) begründet ist.

Bei einer Produktion von 2409,5 Tonnen Rohmethanol pro Tag (t/d) ist im Beispiel der Figur 3 eine Gesamtkompressorleistung von 12,7 für K1 und K2 erforderlich. Die Gesamtkompressorleistung ist damit signifikant niedriger als in den Vergleichsbeispiel gemäß Figur 1 und Figur 2. Bei gleicher Methanol Produktionsmenge ist die Kompressorleistung gemäß dem erfindungsgemäßen setup der Figur 3 um 2,2 MW niedriger als im Vergleichsbeispiel der Figur 1, was einer Einsparung von etwa 15 Prozent entspricht. Gegenüber dem Vergleichsbeispiel der Figur 2 wird immer noch eine Einsparung von 1,0 MW oder etwa 7 Prozent erzielt. Die Einsparung gegenüber dem Vergleichsbeispiel der Figur 2 ist niedriger, jedoch erfordert das setup der Figur 2 insgesamt drei anstatt nur zwei Kompressorstufen. Im Vergleichsbeispiel gemäß Figur 1 und im erfindungsgemäßen Beispiel gemäß Figur 3 ist die Anzahl der Kompressorstufen zwar gleich, jedoch ist die Einsparung gemäß erfindungsgemäßem Beispiel auch deutlich größer im Vergleich zum Vergleichsbeispiel gemäß Figur 2.

In der folgenden Tabelle sind die oben dargestellten Ergebnisse als Gesamtübersicht zusammengefasst.

| | Vergleichsbeispiel 1 (Figur 1) | Vergleichsbeispiel 2 (Figur 2) | Beispiel (Figur 3) |
|---|---|---|---|
| Druck erster Frischgasstrom / bar | 34,0 | 34,0 | 35,0 |
| Druck zweiter Frischgasstrom / bar | n/a | 60,0 | 65,0 |
| Druck Synthesegas (Reaktoreintritt) / bar | 85,0 | 85,0 | 85,0 |
| Druck Reaktoraustritt / bar | 78,0 | 78,0 | 67,0 |
| Anteil Wasserstoff im Frischgas / mol-% | 68,9 | 68,9 | 68,9 |
| Anteil Kohlenmonoxid im Frischgas / mol-% | 18,9 | 18,9 | 18,9 |
| Anteil Kohlendioxid im Frischgas / mol-% | 10,5 | 10,5 | 10,5 |
| Inerte Anteil | Rest | Rest | Rest |

(fortgesetzt)

| | Vergleichsbeispiel 1 (Figur 1) | Vergleichsbeispiel 2 (Figur 2) | Beispiel (Figur 3) |
|---|---|---|---|
| Stöchiometriezahl SN | 2,00 | 2,00 | 2,00 |
| Katalysatorvolumen / $m^3$ | 115 | 115 | 115 (4 × 28,75) |
| Stöchiometriezahl SN | 2,00 | 2,00 | 2,00 |
| Katalysatorvolumen / $m^3$ | 115 | 115 | 115 (4 × 28,75) |
| Methanolproduktion (Rohmethanol) / t/d | 2410 | 2410 | 2409,5 |
| $CO_X$ Umsatz (per pass) / % | 54,5 | 54,5 | 93,9 |
| Kohlenstoff Effizienz / % | 99,1 | 99,1 | 98,8 |
| Rezirkulationrate (Rückführgas/Frischgas) / kmol/kmol | 2,4 | 2,4 | 0,45 |
| Leistung K1 + K2 / MW | 12,5 (K1) | 11,3 (K1+K2) | 12,7 (K1+K2) |
| Leistung RK / MW | 2,4 | 2,4 | - |
| Kompressorleistung gesamt / MW | 14,9 | 13,7 | 12,7 |

Ausführungsformen der Erfindung werden unter Bezugnahme auf verschiedene Arten von Gegenständen beschrieben. Insbesondere werden bestimmte Ausführungsformen unter Bezugnahme auf Ansprüche des Verfahrenstyps beschrieben, während andere Ausführungsformen unter Bezugnahme auf die Ansprüche des Vorrichtungstyps beschrieben werden. Ein Fachmann wird jedoch aus der obigen und der folgenden Beschreibung entnehmen, dass, außer anders angegeben, zusätzlich zu irgendeiner Kombination von Merkmalen, die zu einer Art von Anspruchstyp gehören, auch jede Kombination von Merkmalen in Bezug auf verschiedene Arten von Gegenständen oder Anspruchstypen in Betracht gezogen werden kann.

Während die Erfindung im Detail in der Zeichnung und der vorhergehenden Beschreibung dargestellt und beschrieben wurde, sollen eine solche Darstellung und Beschreibung als veranschaulichend oder beispielhaft und nicht einschränkend betrachtet werden. Die Erfindung ist nicht auf die offenbarten Ausführungsformen beschränkt, sondern lediglich auf die Ansprüche.

In den Ansprüchen schließt das Wort "aufweisend" oder "umfassend" weitere Elemente oder Schritte nicht aus, und der unbestimmte Artikel "ein" oder "eine" schließt eine Mehrzahl nicht aus. Bezugszeichen in den Ansprüchen sollten nicht so ausgelegt werden, dass sie den Umfang der Ansprüche einschränken.

**Bezugszeichenliste**

| | |
|---|---|
| K1 | Erste Kompressorstufe |
| K2 | Zweite Kompressorstufe |
| RK | Rückführgaskompressorstufe |
| R1, R2, R3, R4 | Reaktorstufe, Reaktoreinheit |

| | |
|---|---|
| 101 | Erster Frischgasstrom |
| 102 | Zweiter Frischgasstrom |
| 103 | Synthesegasstrom |
| 104a,b | Rückführgasstrom |
| 105 | nicht reagiertes Synthesegas |
| 106 | Produktstrom (Rohmethanol) |
| 107 | Purgegasstrom |

| | |
|---|---|
| 201 | Erster Frischgasstrom |
| 202 | Zweiter Frischgasstrom |
| 203 | Dritter Frischgasstrom |
| 204 | Synthesegasstrom |
| 205 | nicht reagiertes Synthesegas |
| 206a,b | Rückführgasstrom |
| 207 | Produktstrom (Rohmethanol) |
| 208 | Purgegasstrom |

| | |
|---|---|
| 301 | Erster Frischgasstrom |
| 302 | Zweiter Frischgasstrom |
| 303 | Kombinierter Gasstrom |
| 304 | Synthesegasstrom |
| 305 | Rückführgasstrom |
| 306a,b,c,d | nicht reagiertes Synthesegas |
| 307 | Gesamtproduktstrom (Rohmethanol) |
| 307a,b,c,d | Produktstrom (Rohmethanol) |
| 308 | Purgegasstrom |

**Patentansprüche**

**1.** Verfahren zur Herstellung von Methanol, umfassend folgende Prozessschritte:

a) Bereitstellen eines Kohlenstoffoxide (CO, $CO_2$) und Wasserstoff ($H_2$) aufweisenden Einsatzgases;
b) Einschleusen des Einsatzgases als einen ersten Frischgasstrom in eine erste Kompressorstufe zur Vorverdichtung des ersten Frischgasstroms, wobei ein zweiter Frischgasstrom erhalten wird;
c) Einschleusen eines Rückführgasstroms und des zweiten Frischgasstroms in eine zweite Kompressorstufe zur Verdichtung des Rückführgasstroms und des zweiten Frischgasstroms auf Synthesedruck, wobei ein Synthesegasstrom erhalten wird;
d) Katalytisches Umsetzen des Synthesegases des Synthesegasstroms in einer Mehrzahl in Serie angeordneter Reaktorstufen bei Synthesedruck, wobei je Reaktorstufe ein Methanol und nicht reagiertes Synthesegas aufweisender Produktstrom erhalten wird;
e) Kühlung des je Reaktorstufe erhaltenen Produktstroms zur Kondensation und Abtrennung von Methanol von nicht reagiertem Synthesegas und Einschleusen von nicht reagiertem Synthesegas in eine jeweils folgende der in Serie angeordneten Reaktorstufen;
f) Abziehen von nicht reagiertem Synthesegas aus zumindest einer der Reaktorstufen als Rückführgasstrom zum Einschleusen des Rückführgasstroms in die zweite Kompressorstufe gemäß Schritt c).

**2.** Verfahren nach Anspruch 1, **dadurch gekennzeichnet, dass** der Anteil an Kohlenmonoxid (CO) im Einsatzgas, in Bezug auf die Gesamtmenge der Kohlenstoffoxide, mehr als 20 Vol.-% beträgt, bevorzugt mehr als 50 Vol.-% beträgt, besonders bevorzugt mehr als 75 Vol.-% beträgt und weiter bevorzugt mehr als 90 Vol.-% beträgt.

**3.** Verfahren nach Anspruch 1 oder 2, **dadurch gekennzeichnet, dass** der Rückführgasstrom nach dem Abziehen gemäß Schritt f) ohne vorhergehende Verdichtung in die zweite Kompressorstufe eingeschleust wird.

**4.** Verfahren nach einem der Ansprüche 1 bis 3, **dadurch gekennzeichnet, dass** gemäß Schritt e) nicht reagiertes Synthesegas vollständig in die jeweils folgende der in Serie angeordneten Reaktorstufen eingeschleust wird.

**5.** Verfahren nach einem der vorherigen Ansprüche, **dadurch gekennzeichnet, dass** gemäß Schritt f) nicht reagiertes Synthesegas aus der letzten der Mehrzahl in Serie angeordneten Reaktorstufen als Rückführgasstrom abgezogen wird, insbesondere ausschließlich aus der letzten der Mehrzahl in Serie angeordneten Reaktorstufen abgezogen wird.

**6.** Verfahren nach einem der vorherigen Ansprüche, **dadurch gekennzeichnet, dass** der Rückführgasstrom und der zweite Frischgasstrom zusammengeführt und als kombinierter Gasstrom in die zweite Kompressorstufe eingeschleust werden.

7. Verfahren nach einem der vorherigen Ansprüche, **dadurch gekennzeichnet, dass** das Einsatzgas eine Stöchiometriezahl SN, wobei gilt

$$SN = \frac{n(H_2) - n(CO_2)}{n(CO) + n(CO_2)}\ , mit\ n\ in\ [mol],$$

von 1,5 bis 3,0 aufweist.

8. Verfahren nach einem der vorherigen Ansprüche, **dadurch gekennzeichnet, dass** für eine Rezirkulationsrate R, definiert als

$$R = \frac{Volumenstrom\ (Rückführgasstrom)}{Volumenstrom\ (zweiter\ Frischgasstrom)},$$

$0{,}15 \leq R \leq 1{,}25$ gilt.

9. Verfahren nach Anspruch 8, **dadurch gekennzeichnet, dass** für die Rezirkulationsrate R gilt: $0{,}1 \leq R \leq 0{,}5$

10. Verfahren nach einem der vorherigen Ansprüche, **dadurch gekennzeichnet, dass** über die Gesamtheit der Mehrzahl der in Serie angeordneten Reaktorstufen, ausgehend vom Einsatzgas, ein Kohlenstoffoxide-Umsatz von wenigstens 80 mol-% erzielt wird, bevorzugt von mindestens 90 mol-% erzielt wird, besonders bevorzugt von mindestens 95 mol-% erzielt wird.

11. Verfahren nach einem der vorherigen Ansprüche, **dadurch gekennzeichnet, dass** die Mehrzahl der in Serie angeordneten Reaktorstufen eine Anzahl von 2 bis 8 Reaktorstufen umfasst.

12. Anlage für die Herstellung von Methanol, aufweisend folgende miteinander in Fluidverbindung stehende Anlagenelemente:

Eine erste Kompressorstufe zum Vorverdichten eines Kohlenstoffoxide und Wasserstoff ($H_2$) aufweisenden ersten Frischgases zur Erzeugung eines zweiten Frischgases;
eine stromabwärts zur ersten Kompressorstufe angeordnete zweite Kompressorstufe zur Verdichtung des zweiten Frischgases und eines Rückführgases zu einem Synthesegas bei Synthesedruck;
eine stromabwärts zur zweiten Kompressorstufe angeordnete Mehrzahl in Serie angeordneter Reaktoreinheiten zur Erzeugung eines Methanol und nicht reagiertes Synthesegas aufweisenden Produktstroms aus Synthesegas, wobei jede der Reaktoreinheiten

- einen ein Katalysatorbett aufweisenden Reaktor,
- einen Wärmeaustauscher zur Kühlung des Produktstroms, sowie
- einen Abscheider zur Abtrennung von Methanol von nicht reagiertem Synthesegas

umfasst, wobei
jede der Reaktoreinheiten einen Auslass zum Abziehen von Methanol umfasst und
jede der Reaktoreinheiten einen Auslass zum Zuführen von nicht reagiertem Synthesegas zu einer jeweils nachfolgend angeordneten Reaktoreinheit und/oder
einen Auslass zum Zuführen von nicht reagiertem Synthesegas als Rückführgas zur zweiten Kompressorstufe umfasst, wobei
eine Zuführung zum Einschleusen des Rückführgases in die zweite Kompressorstufe zwischen der ersten und zweiten Kompressorstufe angeordnet ist.

13. Anlage nach Anspruch 12, **dadurch gekennzeichnet, dass** zwischen dem Auslass zum Zuführen von nicht reagiertem Synthesegas als Rückführgas zur zweiten Kompressorstufe und der Zuführung zum Einschleusen des Rückführgases in die zweite Kompressorstufe keine Kompressorstufe zur vorherigen Verdichtung des Rückführgases vor dem Einschleusen in die zweite Kompressorstufe angeordnet ist.

**14.** Anlage nach einem der Ansprüche 12 oder 13, **dadurch gekennzeichnet, dass** ein Auslass zum Zuführen von nicht reagiertem Synthesegas als Rückführgas zur zweiten Kompressorstufe an der letzten der Mehrzahl in Serie angeordneten Reaktoreinheiten angeordnet ist, vorzugsweise ausschließlich an der letzten der Mehrzahl in Serie angeordneten Reaktoreinheiten angeordnet ist.

**15.** Verwendung der Anlage nach einem der Ansprüche 12 bis 14 in einem Verfahren nach einem der Ansprüche 1 bis 11.

## Claims

**1.** Process for producing methanol, comprising the following process steps:

a) providing an input gas comprising carbon oxides (CO, $CO_2$) and hydrogen ($H_2$);
b) introducing the input gas as a first fresh gas stream into a first compressor stage for precompression of the first fresh gas stream to obtain a second fresh gas stream;
c) introducing a recycle gas stream and the second fresh gas stream into a second compressor stage for compression of the recycle gas stream and the second fresh gas stream to synthesis pressure to obtain a synthesis gas stream;
d) catalytically converting the synthesis gas of the synthesis gas stream in a plurality of serially arranged reactor stages at synthesis pressure to obtain a product stream comprising methanol and unreacted synthesis gas per reactor stage;
e) cooling the product stream obtained per reactor stage for condensation and separation of methanol from unreacted synthesis gas and introducing unreacted synthesis gas into a respective subsequent stage of the serially arranged reactor stages;
f) withdrawing unreacted synthesis gas from at least one of the reactor stages as a recycle gas stream for introduction of the recycle gas stream into the second compressor stage according to step c).

**2.** Process according to Claim 1, **characterized in that** the proportion of carbon monoxide (CO) in the input gas, based on the total amount of the carbon oxides, is more than 20% by volume, preferably more than 50% by volume, particularly preferably more than 75% by volume and more preferably more than 90% by volume.

**3.** Process according to Claim 1 or 2, **characterized in that** after the withdrawing according to step f) the recycle gas stream is introduced into the second compressor stage without preceding compression.

**4.** Process according to any of Claims 1 to 3, **characterized in that** according to step e) unreacted synthesis gas is completely introduced into the respective subsequent stage of the serially arranged reactor stages.

**5.** Process according to any of the preceding claims, **characterized in that** according to step f) unreacted synthesis gas is withdrawn as a recycle gas stream from the last of the plurality of serially arranged reactor stages, in particular exclusively from the last of the plurality of serially arranged reactor stages.

**6.** Process according to any of the preceding claims, **characterized in that** the recycle gas stream and the second fresh gas stream are merged and introduced into the second compressor stage as a combined gas stream.

**7.** Process according to any of the preceding claims, **characterized in that** the input gas has a stoichiometry number SN of 1.5 to 3.0, wherein

$$SN = \frac{n(H_2) - n(CO_2)}{n(CO) + n(CO_2)}, with\ n\ in\ [mol].$$

**8.** Process according any of the preceding claims, **characterized in that** for a recirculation rate R defined as

$$R = \frac{Volume\ flow\ (recycle\ gas\ stream)}{Volume\ flow\ (second\ fresh\ gas\ stream)},$$

$$0.15 \leq R \leq 1.25.$$

**9.** Process according to Claim 8, **characterized in that** for the recirculation rate R: $0.1 \leq R \leq 0.5$.

**10.** Process according to any of the preceding claims, **characterized in that** based on the input gas a carbon oxides conversion of at least 80 mol% is achieved, preferably of at least 90 mol% is achieved, particularly preferably of at least 95 mol% is achieved, over the entirety of the plurality of serially arranged reactor stages.

**11.** Process according to any of the preceding claims, **characterized in that** the plurality of serially arranged reactor stages comprises a number of 2 to 8 reactor stages.

**12.** Plant for producing methanol comprising the following plant elements arranged in fluid connection with one another:

a first compressor stage for precompression of a first fresh gas comprising carbon oxides and hydrogen ($H_2$) to produce a second fresh gas;
a second compressor stage for compression of the second fresh gas and a recycle gas to afford a synthesis gas at synthesis pressure arranged downstream of the first compressor stage;
a plurality of serially arranged reactor units for producing a product stream comprising methanol and unreacted synthesis gas from synthesis gas arranged downstream of the second compressor stage, wherein each of the reactor units comprises

- a reactor comprising a catalyst bed,
- heat exchanger for cooling the product stream and
- a separator for separating methanol from unreacted synthesis gas,

wherein

each of the reactor units comprises an outlet for withdrawing methanol and
each of the reactor units comprises an outlet for supplying unreacted synthesis gas to a respective downstream reactor unit and/or
an outlet for supplying unreacted synthesis gas as recycle gas to the second compressor stage, wherein
a feed for introducing the recycle gas into the second compressor stage is arranged between the first and second compressor stage.

**13.** Plant according to Claim 12, **characterized in that** no compressor stage for precompression of the recycle gas before introduction into the second compressor stage is arranged between the outlet for supplying unreacted synthesis gas to the second compressor stage as recycle gas and the feed for introducing the recycle gas into the second compressor stage.

**14.** Plant according to either of Claims 12 and 13, **characterized in that** an outlet for supplying unreacted synthesis gas to the second compressor stage as recycle gas is arranged on the last of the plurality of serially arranged reactor units, preferably exclusively on the last of the plurality of serially arranged reactor units.

**15.** Use of the plant according to any of Claims 12 to 14 in a process according to any of Claims 1 to 11.

**Revendications**

**1.** Procédé de production de méthanol, ledit procédé comprenant les étapes de processus suivantes :

a) fournir un gaz d'alimentation contenant des oxydes de carbone ($CO$, $CO_2$) et de l'hydrogène ($H_2$) ;
b) injecter le gaz d'alimentation comme premier flux de gaz frais dans un premier étage de compresseur afin de pré-comprimer le premier flux de gaz frais, un deuxième flux de gaz frais étant obtenu ;
c) injecter un flux de gaz de recyclage et le deuxième flux de gaz frais dans un deuxième étage de compresseur afin de comprimer le courant de gaz de recyclage et le deuxième flux de gaz frais à la pression de synthèse, un flux de gaz de synthèse étant obtenu ;
d) effectuer une conversion catalytique du gaz de synthèse du flux de gaz de synthèse dans une pluralité

d'étages de réacteur disposés en série à la pression de synthèse, un flux de produit contenant du méthanol et du gaz de synthèse n'ayant pas réagi étant obtenu à chaque étage du réacteur ;
e) refroidir le flux de produit obtenu à chaque étage de réacteur afin de condenser et d'éliminer le méthanol du gaz de synthèse n'ayant pas réagi et injecter le gaz de synthèse n'ayant pas réagi dans un étage de réacteur respectivement suivant parmi les étages de réacteur disposés en série ;
f) retirer le gaz de synthèse n'ayant pas réagi d'au moins un des étages de réacteur comme flux de gaz de recyclage afin d'injecter le flux de gaz de recyclage dans le deuxième étage de compresseur selon l'étape c).

**2.** Procédé selon la revendication 1, **caractérisé en ce que** la proportion de monoxyde de carbone (CO) dans le gaz d'alimentation, rapportée à la quantité totale d'oxydes de carbone, est supérieure à 20 % en volume, de préférence supérieure à 50 % en volume, de manière particulièrement préférée supérieure à 75 % en volume et plus préférablement supérieure à 90 % en volume.

**3.** Procédé selon la revendication 1 ou 2, **caractérisé en ce que** le flux de gaz de recyclage est injecté dans le deuxième étage du compresseur sans compression préalable après avoir été prélevé selon l'étape f).

**4.** Procédé selon l'une des revendications 1 à 3, **caractérisé en ce que** selon l'étape e) le gaz de synthèse n'ayant pas réagi est injecté en totalité dans l'étage de réacteur respectivement suivant parmi les étages de réacteur disposés en série.

**5.** Procédé selon l'une des revendications précédentes, **caractérisé en ce que** selon l'étape f) le gaz de synthèse n'ayant pas réagi est prélevé comme flux de gaz de recyclage sur le dernier étage de réacteur de la pluralité d'étages de réacteur disposés en série, en particulier est prélevé exclusivement du dernier étage de réacteur parmi la pluralité d'étages de réacteur disposés en série.

**6.** Procédé selon l'une des revendications précédentes, **caractérisé en ce que** le flux de gaz de recyclage et le deuxième flux de gaz frais sont réunis et injectés comme flux de gaz combiné dans le deuxième étage de compresseur.

**7.** Procédé selon l'une des revendications précédentes, **caractérisé en ce que** le gaz d'alimentation a un nombre de stœchiométrie SN allant de 1,5 à 3,0, avec

$$SN = \frac{n(H_2) - n(CO_2)}{n(CO) + n(CO_2)}$$

et n en [mol].

**8.** Procédé selon l'une des revendications précédentes, **caractérisé en ce que** pour un taux de recirculation R, défini par

$$R = \frac{\text{flux en volume (flux de gaz de recyclage)}}{\text{flux en volume (deuxième flux de gaz frais)}}$$

avec $0{,}15 \leq R \leq 1{,}25$

**9.** Procédé selon la revendication 8, **caractérisé en ce que** pour un taux de recirculation R on a : $0{,}1 \leq R \leq 0{,}5$.

**10.** Procédé selon l'une des revendications précédentes, **caractérisé en ce que**, sur l'ensemble de la pluralité d'étages de réacteur disposés en série, une conversion d'oxyde de carbone d'au moins 80 % molaires, de préférence d'au moins 90 % molaires, plus préférablement d'au moins 95 % molaires est obtenue à partir du gaz d'alimentation.

**11.** Procédé selon l'une des revendications précédentes, **caractérisé en ce que** la pluralité d'étages de réacteur disposés en série comprend un nombre de 2 à 8 étages de réacteur.

**12.** Installation de production de méthanol, comportant les éléments d'installation suivants qui sont en communication fluidique les uns avec les autres :

un premier étage de compresseur destiné à pré-comprimer un premier gaz frais contenant des oxydes de carbone et
de l'hydrogène ($H_2$) afin de produire un deuxième gaz frais ;
un deuxième étage de compresseur disposé en aval du premier étage de compresseur pour comprimer le deuxième gaz frais et un gaz de recyclage afin d'obtenir un gaz de synthèse à la pression de synthèse ;
une pluralité d'unités de réacteur disposées en série, laquelle pluralité est disposée en aval du deuxième étage de compresseur, pour produire à partir du gaz de synthèse un flux de produit contenant du méthanol et du gaz de synthèse n'ayant pas réagi, chacune des unités de réacteur comprenant

- un réacteur pourvu d'un lit catalytique,
- un échangeur de chaleur destiné à refroidir le flux de produit, et
- un séparateur destiné à séparer le méthanol du gaz de synthèse n'ayant pas réagi,

chacune des unités de réacteur comprenant une sortie destiné à prélever du méthanol et
chacune des unités de réacteur comprenant une sortie destinée à fournir du gaz de synthèse n'ayant pas réagi à une unité de réacteur respectivement en aval et/ou une sortie destinée à fournir du gaz de synthèse n'ayant pas réagi comme gaz de recyclage au deuxième étage de compresseur,
une alimentation destinée à injecter le gaz de recyclage dans le deuxième étage de compresseur étant disposée entre le premier et le deuxième étage de compresseur.

**13.** Installation selon la revendication 12, **caractérisée en ce qu'**aucun étage de compresseur destiné à la compression préalable du gaz de recyclage avant d'injecter celui-ci dans le deuxième étage de compresseur n'est disposé entre la sortie d'alimentation du deuxième étage de compresseur en gaz de synthèse n'ayant pas réagi comme gaz de recyclage et l'alimentation destinée à injecter le gaz de recyclage dans le deuxième étage de compresseur.

**14.** Installation selon l'une des revendications 12 et 13, **caractérisée en ce qu'**une sortie d'alimentation du deuxième étage de compresseur en gaz de synthèse n'ayant pas réagi comme gaz de recyclage est disposée au niveau de la dernière unité de réacteur parmi la pluralité d'unités de réacteur disposées en série, de préférence exclusivement au niveau de la dernière unité de réacteur parmi la pluralité d'unités de réacteur disposées en série.

**15.** Utilisation de l'installation selon l'une des revendications 12 à 14 dans un procédé selon l'une des revendications 1 à 11.

Fig. 1

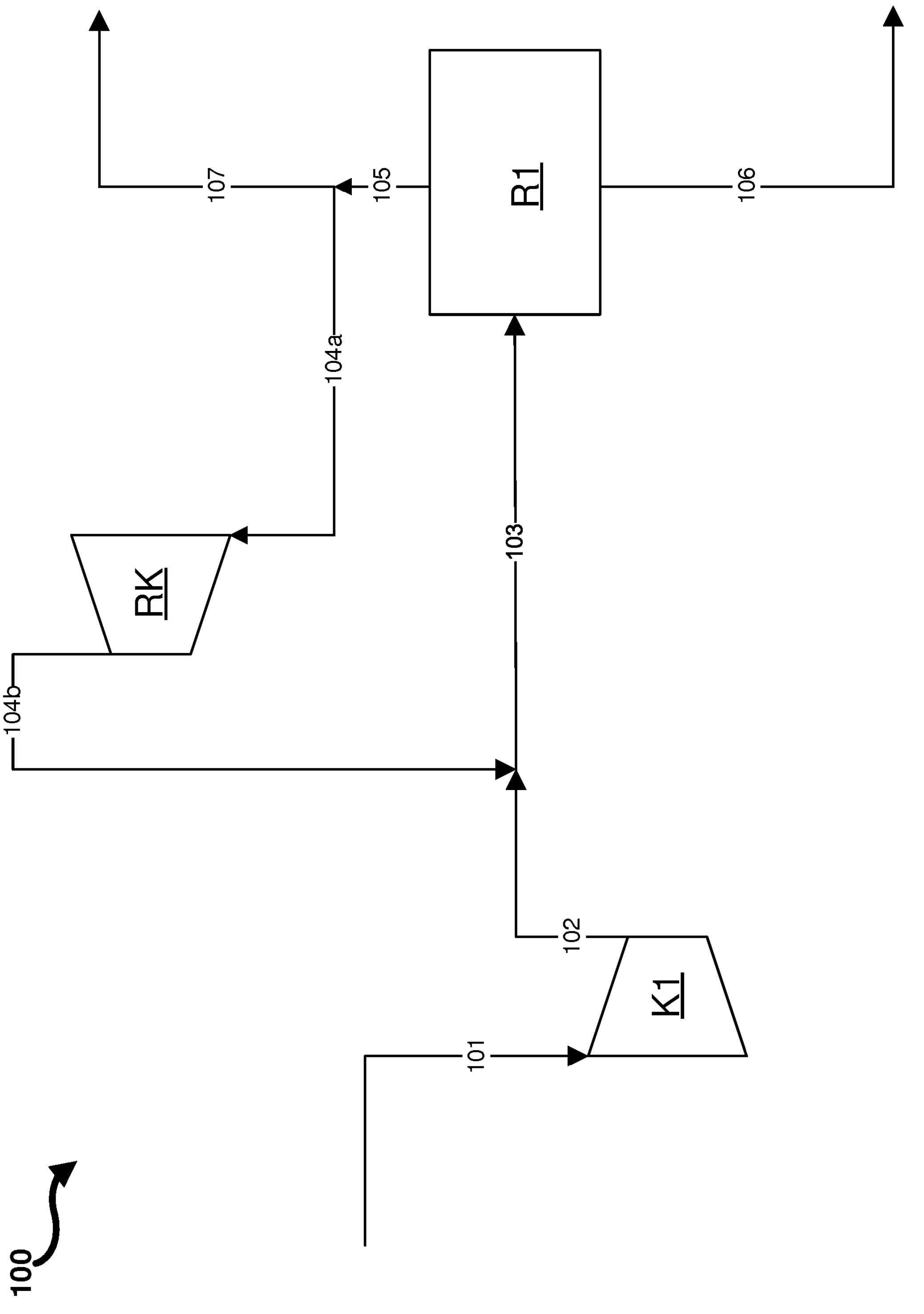

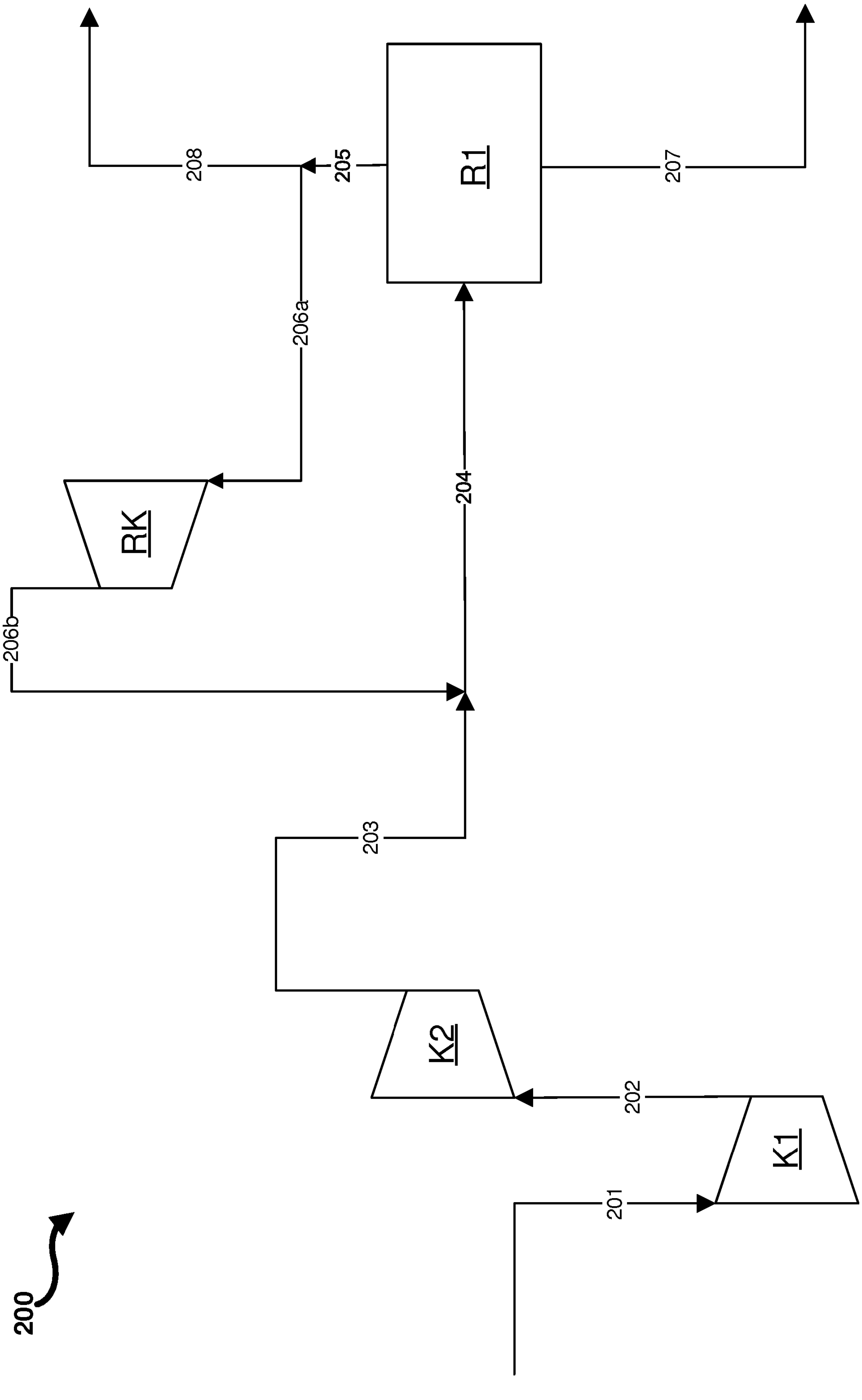


Fig. 2

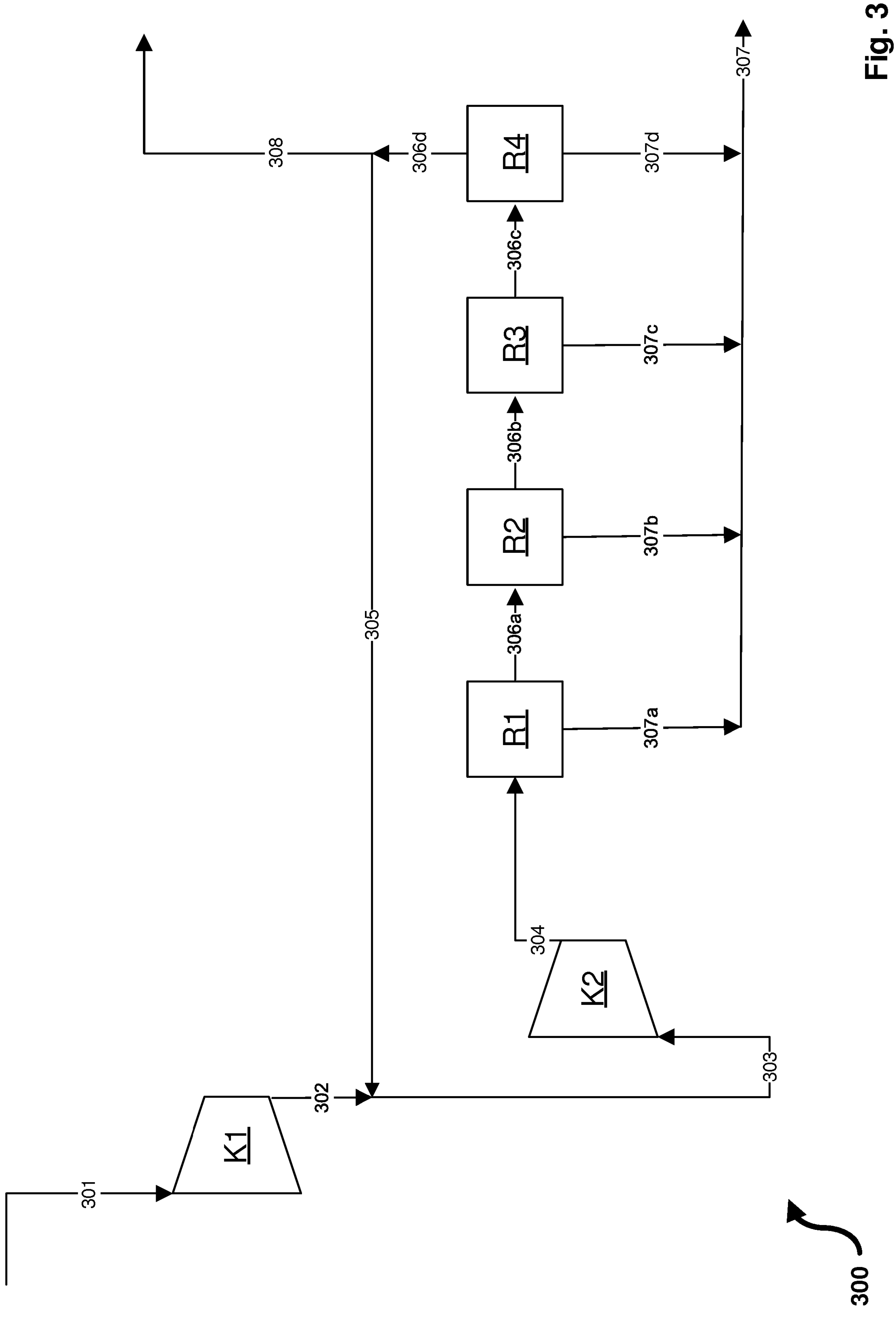
301
K1
302
303
K2
304
305
R1
306a
R2
306b
R3
306c
R4
306d
307a
307b
307c
307d
307
308
300

Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2015193440 A1
- WO 2017167642 A1
- EP 2116295 A1